# EUROPEAN PATENT APPLICATION

(11) **EP 1 958 658 A1**
(43) Date of publication of application: **20.08.2008**
(21) Application number: 08002636.2
(22) Date of filing: 13.02.2008
(51) Int. Cl.: A61M 25/10, A61B 1/12

(54) **Medical apparatus with liquid recovering reservoir**

(30) Priority: 16.02.2007 JP 2007036925
(71) Applicant: OLYMPUS MEDICAL SYSTEMS CORP., Shibuya-ku Tokyo 151-0072 (JP)
(72) Inventor: Niwa, Hiroshi, Hachioji-shi Tokyo 192-8512 (JP); Fukuchi, Masami, Hachioji-shi Tokyo 192-8512 (JP); Karasawa, Isamu, Hachioji-shi Tokyo 192-8512 (JP); Kami, Kuniaki, Hachioji-shi Tokyo 192-8512 (JP); Kishi, Kenji, Hachioji-shi Tokyo 192-8512 (JP); Miyashita, Akihiro, Hachioji-shi Tokyo 192-8512 (JP)
(74) Representative: von Hellfeld, Axel

(57) **Abstract**

In a case where a balloon (26) is broken and a body fluid reversely flows into a feed/discharge tube (28) during a suction operation of a balloon control apparatus (1), a pump (31) is stopped by operation-canceling means after passing of a preset predetermined time (e.g. 20 seconds), and the suction operation of sucking the fluid into a bottle (89) is canceled. Thereby, the amount of recovered liquid in the bottle (89) is limited within a predetermined capacity. Thus, even in the case where the body fluid reversely flows into the feed/discharge tube (28), the body fluid does not overflow from the bottle (89), and the body liquid can properly recovered in the bottle (89).

## Description

The present invention relates to a medical apparatus which includes a pressure source and is used at a time of feeding/discharging air to/from a balloon, which is attached to medical equipment such as an insertion tool for facilitating insertion of an endoscope into a body cavity, thereby inflating/deflating the balloon.

In general, when an insertion section of an endoscope is inserted in a tubular organ within a deep part of the body, such as a small intestine, an insertion tool for facilitating insertion of the insertion section is used. Jpn. Pat. Appln. KOKAI Publication No. 2004-329720 (Patent Document 1) discloses an overtube as the insertion tool. The insertion section of the endoscope is advancibly/retreatably inserted in the overtube. A distal end portion of the overtube and a distal end portion of the insertion section of the endoscope are provided with balloons, respectively. Air is fed/discharged to/from the balloon at the distal end portion of the overtube and the balloon at the distal end portion of the insertion section of the endoscope via separate air supply tubes.

At a time of insertion into the body, the overtube and the insertion section are inserted into the body cavity in the state in which the insertion section is inserted in the overtube. Then, the overtube and the insertion section are alternately advanced. Thereby, the insertion section is inserted into a deeper part of the body cavity. At this time, air is fed/discharged into/from the balloon, where necessary, thus performing an operation of inflating the balloon and engaging the balloon with the inner surface of the body cavity, and an operation of deflating the balloon and disengaging the balloon from the inner surface of the body cavity.

Further, in the above-mentioned Patent Document 1, a reservoir tank is connected to an intermediate portion of the conduit through which air is fed from an air feed/suction device to the balloon and air is discharged from the balloon to the air feed/suction device. In a case where the balloon is broken and a body fluid reversely flows into the conduit, this body fluid is stored in the reservoir tank. Thereby, reverse flow of the body fluid to the air feed/suction device is prevented.

Jpn. Pat. Appln. KOKAI Publication No. 2000-305623 (Patent Document 2) discloses that a suction bottle for recovering a body fluid, etc. is provided in a suction conduit of an endoscope. In this case, a liquid level sensor is provided near the suction bottle. Detection means is disclosed, wherein the height of the liquid level within the suction bottle is sensed by the liquid level sensor and thus the full liquid state of the suction bottle is detected.

In the apparatus of Patent Document 1, in the case where the balloon is punctured and body fluid reversely flows, the body fluid can be stored in the reservoir tank, thereby preventing the body fluid from reversely flowing to the air feed/suction device. However, at the time of a continuous suction operation, there is a case in which the recovered fluid exceeds the capacity of the reservoir tank. Thus, at the time of suction, it is necessary to pay attention so as to prevent such continuous suction as to exceed the capacity of the reservoir tank, and the operation therefor is time-consuming.

In particular, at a time of continuous suction, there is a case in which a relatively large amount of liquid is recovered. It is thus possible that such a suction operation as to exceed the capacity of the reservoir tank is performed. In such a case, the body fluid may reversely flow to the suction pump side of the air feed/suction apparatus.

In the apparatus of Patent Document 2, the detection means, such as a liquid level sensor, needs to be provided near the suction bottle. In the case where the detection means is provided, the structure of the entire apparatus may become complex, and the cost may increase.

The present invention has been made in consideration of the above-described circumstances, and the object of the invention is to provide a medical apparatus which can properly recover a body fluid even when the body fluid reversely flows into a suction conduit, and can suppress an increase in manufacturing cost.

According to a first aspect of the present invention, a medical apparatus comprising: medical equipment which is provided with an opening end for feeding/discharging a liquid; a conduit for feeding/discharging a fluid, which has one end communicating with the opening end of the medical equipment; a pressure source which generates a pressure difference for suction via the conduit; a control unit which controls a suction operation from the opening end via the conduit on the basis of the pressure difference that is generated by the pressure source, in accordance with an instruction from instruction means for instructing at least the suction operation from the opening end of the medical equipment; reservoir means for recovering a liquid that moves in the conduit in accordance with the suction operation based on a control of the control unit, the reservoir means communicating with the conduit and having a preset predetermined capacity for storing the liquid; and operation-canceling means for canceling the suction operation of sucking the liquid into the reservoir means, thereby to limit an amount of the recovered liquid in the reservoir means within the predetermined capacity.

In the above-described structure, when the liquid that moves in the conduit in accordance with the suction operation based on the control of the control unit is recovered in the reservoir means having the predetermined capacity, the operation-canceling means cancels the suction operation of sucking the liquid into the reservoir means. Thereby, the amount of the recovered liquid in the reservoir means is limited within the preset capacity.

Preferably, the medical equipment includes an insertion section which is inserted in a body cavity, the insertion section includes a balloon which inflates/deflates in accordance with the pressure difference, and the conduit permits communication between the pressure source and the balloon.

In the above-described structure, a pressure difference is transmitted to the balloon of the insertion section of the medical equipment via the conduit that permits communication between the pressure source and the balloon, and the balloon is inflated/deflated in accordance with the pressure difference.

Preferably, the operation-canceling means includes at least one of means for stopping the pressure source after passing of a preset predetermined time, means for shutting the conduit after the passing of the preset predetermined time, and means for leak to outside of the conduit after the passing of the preset predetermined time.

In the above-described structure, when the operation-canceling means is activated, at least one of the means for stopping the pressure source after passing of a preset time, the means for shutting the conduit after the passing of the preset time and the means for leak to outside of the conduit after the passing of the preset time is operated.

Preferably, the reservoir means is a bottle for liquid recovery, a main body of the medical apparatus includes a bottle holding section which rotatably holds the bottle, and the bottle is rotatable relative to the bottle holding section in accordance with movement of the conduit.

In the above-described structure, the bottle for liquid recovery of the reservoir means is rotatably held by the bottle holding section of the main body of the medical apparatus. Thereby, the bottle is made rotatable relative to the bottle holding section in accordance with the movement of the conduit, so that the conduit may not become an obstacle.

Preferably, the bottle is configured such that an end face of a connection part for connection to the conduit is disposed at a position which is apart, by a predetermined distance, from a liquid level of the liquid that is recovered in a predetermined time.

In the above-described structure, the end face of the connection part for connection to the conduit is disposed at a position which is apart, by a predetermined distance, from the liquid level of the liquid that is recovered in the bottle in a predetermined time until the operation-canceling means is operated. Thereby, even in the case where the bottle is horizontally fallen or is turned upside down, the end face of the connection part for connection to the conduit is projected away from the liquid level of the liquid, and the liquid recovered in the bottle does not leak.

Preferably, the conduit includes a medical-equipment-side conduit which is connected to an inner conduit of the medical equipment, and a pressure-source-side conduit which is connected to the pressure source, the bottle includes a cylindrical bottle body, the bottle holding section includes a C-shaped bottle holder which holds the bottle body so as to be rotatable about a center axis of the bottle body in a state in which the center axis of the bottle body is set in a horizontal direction, and the bottle body is provided with a connection part for connection to the pressure-source-side conduit at an upper end surface thereof, and a connection part for connection to the medical-equipment-side conduit at an outer peripheral surface thereof.

In the above-described structure, the cylindrical bottle body is held by the C-shaped bottle holder of the bottle holding section so as to be rotatable about the center axis of the bottle body. Thereby, the bottle is made rotatable relative to the bottle holding section in accordance with the movement of the medical-equipment-side conduit that is connected to the connection part on the outer peripheral surface of the bottle body, so that the conduit may not become an obstacle.

Preferably, the main body of the medical apparatus includes a box-shaped outer case which accommodates the pressure source and the control unit, the case includes, on a front surface thereof, a concave-shaped bottle receiving recess portion which receives the bottle, a mount portion of the instruction means, and a led-out portion of the pressure-source-side conduit, and the C-shaped bottle holder is mounted in the bottle receiving recess portion.

In the above-described structure, the box-shaped outer case of the medical apparatus includes, on the front surface thereof, the concave-shaped bottle receiving recess portion, the mount portion of the instruction means, and the led-out portion of the pressure-source-side conduit. The C-shaped bottle holder is mounted in the concave-shaped bottle receiving recess portion so that the bottle can be received in the bottle receiving recess portion.

Preferably, the bottle body is configured such that an inner end portion of the connection part for connection to the pressure-source-side conduit and an inner end portion of the connection part for connection to the medical-equipment-side conduit are positioned above the liquid level of the liquid that is recovered in the bottle body in the predetermined time.

In the above-described structure, the inner end portion of the connection part for connection to the pressure-source-side conduit and the inner end portion of the connection part for connection to the medical-equipment-side conduit are positioned above the liquid level of the liquid that is recovered in the bottle body in the predetermined time until the operation-canceling means is operated. Thereby, leak of the liquid recovered in the bottle is prevented.

Preferably, the medical equipment includes an insertion section of an endoscope, and an overtube which is provided over the insertion section of the endoscope, and the balloon is provided over the overtube.

In the above-described structure, the medical equipment is configured such that the overtube is provided over the insertion section of the endoscope, and the balloon is provided over the overtube.

Preferably, the pressure source is composed of a pump, the pressure-source-side conduit includes at least an air feed flow path which causes a discharge pressure of the pump to act on the bottle side, and a suction flow path which causes a suction pressure of the pump to act on the bottle side, and the control unit includes switching means for effecting switching between the air feed flow path and the suction flow path in accordance with an instruction from the instruction means.

In the above-described structure, the pressure-source-side conduit includes at least the air feed flow path which causes the discharge pressure of the pump to act on the bottle side, and the suction flow path which causes the suction pressure of the pump to act on the bottle side. The air feed flow path and the suction flow path are switched by the switching means of the control unit in accordance with an instruction from the instruction means. Thereby, the single pump can be shared at the time of air feed and at the time of air suction, and the number of pumps can be decreased.

Preferably, the instruction means includes at least a first controller which is fixed to the case of the main body of the medical apparatus, and a second controller which is a remote controller that is connected to the main body of the medical apparatus.

In the above-described structure, the suction operation from the opening end of the medical equipment can be instructed by the two instruction means comprising at least the first controller which is fixed to the case of the main body of the medical apparatus, and the second controller which is the remote controller connected to the main body of the medical apparatus.

Preferably, the instruction means includes a third controller which is a foot switch that is connected to the main body of the medical apparatus.

In the above-described structure, the suction operation from the opening end of the medical equipment can also be instructed by the instruction means of the third controller which is the foot switch that is connected to the main body of the medical apparatus.

Preferably, the pressure-source-side conduit includes a relief valve which is held in a closed state when a pressure in the conduit is lower than a preset pressure, and is opened when the pressure in the conduit becomes higher than the preset pressure, and the relief valve is disposed at a position which is closest to the bottle side in the pressure-source-side conduit.

In the above-described structure, the pressure-source-side conduit is provided with the mechanical relief valve which is held in the closed state when the pressure in the conduit is lower than the preset pressure, and is opened when the pressure in the conduit becomes higher than the preset pressure. Thereby, the relief valve is not subjected to electronic control and is opened by only the variation in the pressure in the conduit pressure. In addition, the relief valve is disposed at a position which is closest to the bottle side in the pressure-source-side conduit, and thereby the safety can be enhanced.

According to another aspect of the present invention, a medical apparatus comprising: medical equipment which is provided with an opening end for feeding/discharging a liquid; a conduit for feeding/discharging a fluid, which has one end communicating with the opening end of the medical equipment; a pressure source which generates a pressure difference for suction via the conduit; a control unit which controls a suction operation from the opening end via the conduit on the basis of the pressure difference that is generated by the pressure source, in accordance with an instruction from instruction unit for instructing at least the suction operation from the opening end of the medical equipment; a bottle for recovering a liquid that moves in the conduit in accordance with the suction operation based on a control of the control unit, the bottle communicating with the conduit and having a preset predetermined capacity for storing the liquid; and operation-canceling section for canceling the suction operation of sucking the liquid into the bottle, thereby to limit an amount of the recovered liquid in the bottle within the predetermined capacity.

The present invention can provide a medical apparatus which can properly recover a body fluid even when the body fluid reversely flows into a suction conduit, and can suppress an increase in manufacturing cost.

The invention can be more fully understood from the following detailed description when taken in conjunction with the accompanying drawings, in which:
FIG. 1 schematically shows the entire structure of an endoscope system in which a balloon control apparatus, which is a medical apparatus according to a first embodiment of the present invention, is assembled;
FIG. 2 shows an endoscope and an insertion tool in the first embodiment;
FIG. 3 is a side view showing a state in which the endoscope is inserted and assembled in the insertion tool in the first embodiment;
FIG. 4 is a perspective view showing a state of connection between the balloon control apparatus of the first embodiment and a medical-equipment-side conduit of the insertion tool;
FIG. 5 is a perspective view showing a state of the balloon control apparatus before a liquid recovery bottle is mounted on the balloon control apparatus of the first embodiment;
FIG. 6 is a front view of the balloon control apparatus, showing a state in which the liquid recovery bottle is mounted on the balloon control apparatus of the first embodiment;
FIG. 7 is a perspective view showing a state at a time of use of the balloon control apparatus of the first embodiment;
FIG. 8 is a longitudinal cross-sectional view of the liquid recovery bottle of the balloon control apparatus of the first embodiment;
FIG. 9 is a longitudinal cross-sectional view showing a state in which the liquid recovery bottle of the balloon control apparatus of the first embodiment is horizontally situated;
FIG. 10 is a view for explaining a rotational state of the liquid recovery bottle of the balloon control apparatus according to the first embodiment;
FIG. 11 schematically shows the entire configuration of pressure-source-side conduits of the balloon control apparatus of the first embodiment;
FIG. 12 is a schematic structural view showing a control unit of the balloon control apparatus of the first embodiment;
FIG. 13A is a longitudinal cross-sectional view showing a state in which no power is supplied to a solenoid of a pinch valve which is assembled in the pressure-source-side conduit of the balloon control apparatus of the first embodiment;
FIG. 13B is a longitudinal cross-sectional view showing a state in which power is supplied to the solenoid;
FIG. 14A is a characteristic view showing an open/closed state of a conduit by the pinch valve of the balloon control apparatus of the first embodiment;
FIG. 14B is a characteristic graph showing a relationship between a ratio of an opening/closing time of the conduit by the pinch valve and an air flow amount;
FIG. 15A is a longitudinal cross-sectional view showing a state in which a relief valve of the balloon control apparatus of the first embodiment is closed;
FIG. 15B is a longitudinal cross-sectional view showing a state in which the relief valve is opened;
FIG. 16 schematically shows the entire configuration of the pressure-source-side conduits of the balloon control apparatus of the first embodiment at a time of air feed;
FIG. 17 schematically shows the entire configuration of the pressure-source-side conduits of the balloon control apparatus of the first embodiment at a time of air suction;
FIG. 18 schematically shows the entire configuration of the pressure-source-side conduits of the balloon control apparatus of the first embodiment at a time of conduit hold;
FIG. 19 schematically shows the entire configuration of the pressure-source-side conduits of the balloon control apparatus of the first embodiment at a time of opening to outside which is effected by power-off;
FIG. 20 schematically shows the entire configuration of the pressure-source-side conduits of the balloon control apparatus of the first embodiment at a time of abnormality in conduit pressure due to a failure of a pressure sensor;
FIG. 21 schematically shows the entire configuration of the pressure-source-side conduits of the balloon control apparatus of the first embodiment at a time of abnormality in conduit pressure due to a failure of the valve;
FIG. 22 is a flow chart for describing an operation state of a main operation of the balloon control apparatus of the first embodiment;
FIG. 23 is a flow chart for describing an operation state at a time of an air feed process of the balloon control apparatus of the first embodiment;
FIG. 24 is a flow chart for describing an operation state at a time of an air suction process of the balloon control apparatus of the first embodiment;
FIG. 25 is a flow chart for describing an operation state at a time of an inspection mode of the balloon control apparatus of the first embodiment;
FIG. 26 is a flow chart for describing an operation state at a time of an error process of the balloon control apparatus of the first embodiment;
FIG. 27 is a front view showing a remote controller of a balloon control apparatus according to a second embodiment of the present invention;
FIG. 28 is a perspective view showing a bottle holding section of a balloon control apparatus according to a third embodiment of the present invention;
FIG. 29A is a side view of the bottle holding section of the balloon control apparatus according to the third embodiment; and
FIG. 29B is a plan view of the bottle holding section.

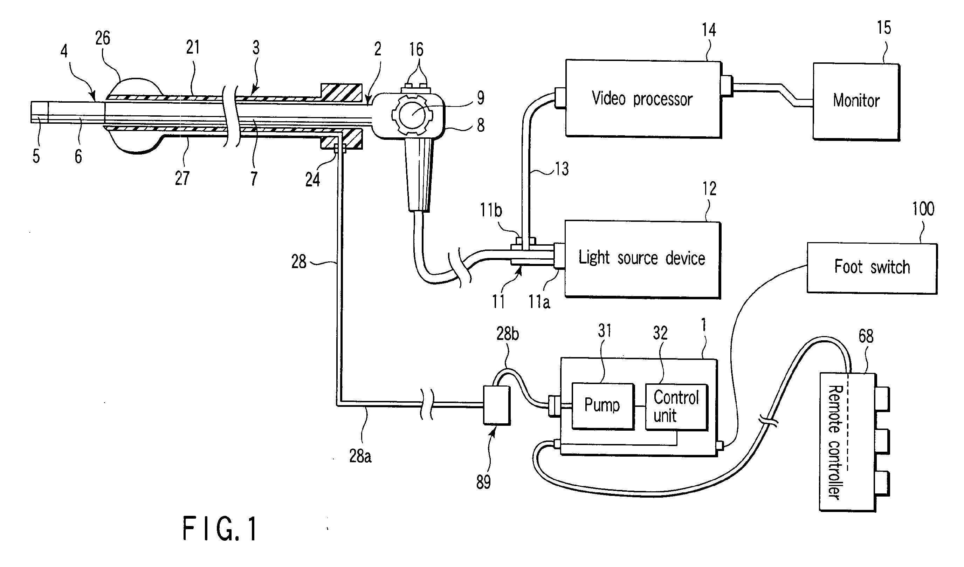

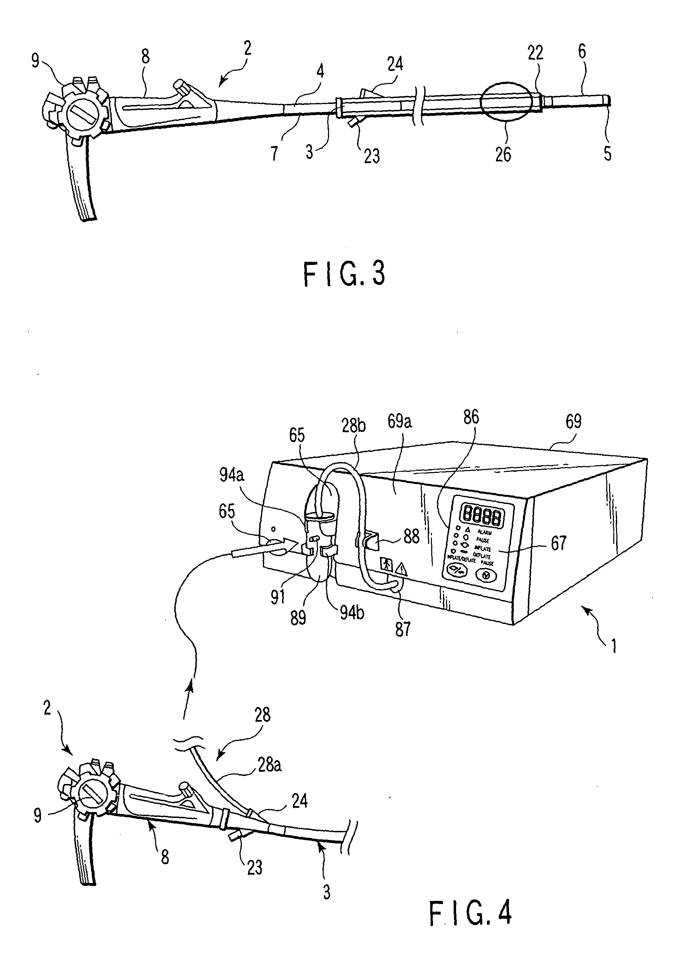

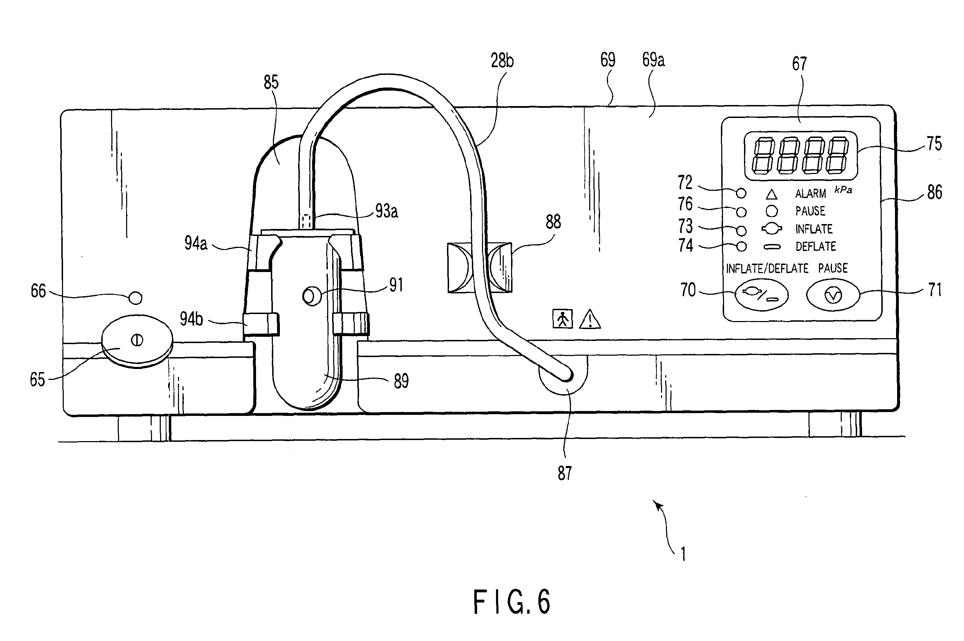

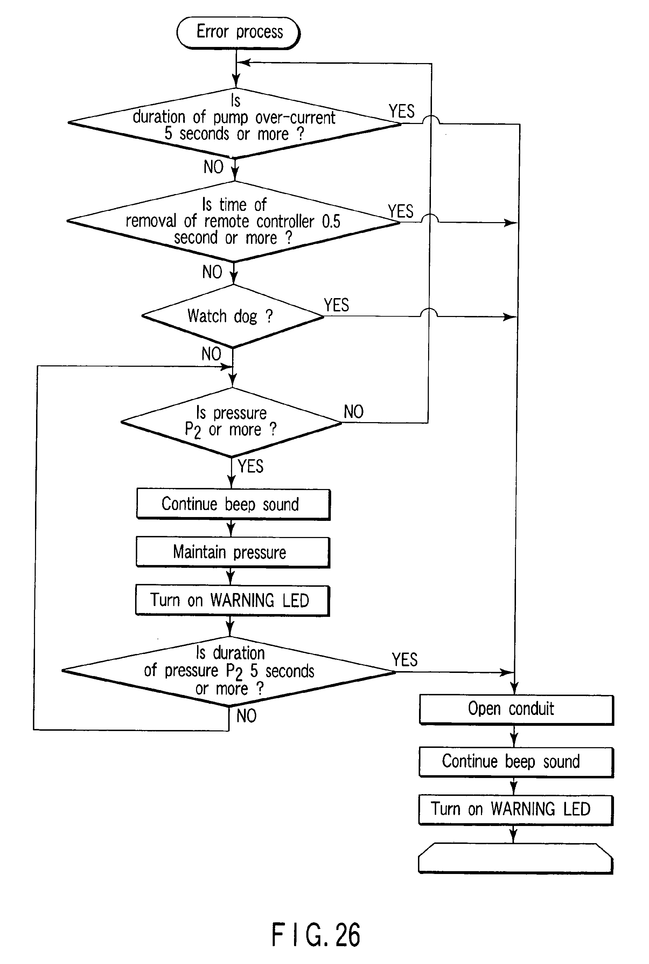

A first embodiment of the present invention will now be described with reference to FIG. 1 to FIG. 26. FIG. 1 schematically shows the entire structure of an endoscope system in which a balloon control apparatus, which is a medical apparatus according to the present embodiment, is assembled.

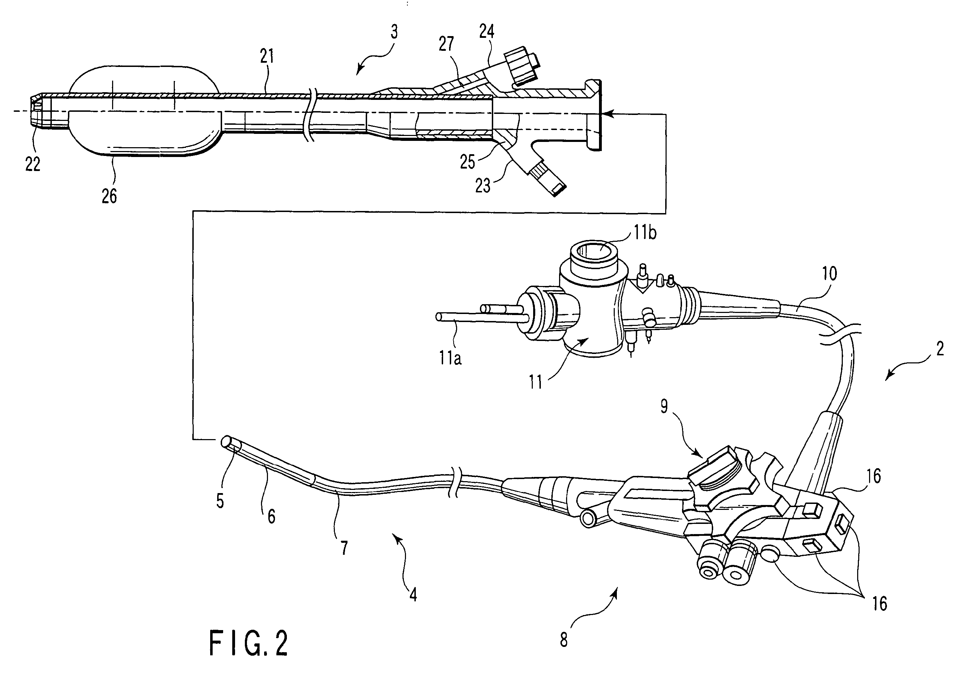

In FIG. 1 and FIG. 2, reference numeral 2 denotes an endoscope, and numeral 3 denotes an overtube which serves as an insertion tool for facilitating insertion of the endoscope 2. The endoscope 2 includes an elongated insertion section 4 which is inserted into a body cavity. The insertion section 4 includes a distal-end rigid section 5, a bending section 6 is bend-operated, and a flexible tube section 7 having an elongated shape and flexibility. The distal-end rigid section 5, bending section 6 and flexible tube section 7 are successively coupled from the distal end side, and thereby the insertion section 4 is constituted.

A proximal end portion of the insertion section 4 is coupled to an operation section 8 which is held and operated by an operator. The operation section 8 is provided with, for instance, a bend operation knob 9 for bend-operating the bending section 6. A universal cord 10 extends from the operation section 8. A connector section 11 is provided at an extension end portion of the universal cord 10. The connector section 11 includes a light source connector 11a and an electric connector 11b. The light source connector 11a is connected to a light source device 12. Illumination light from the light source device 12 is transmitted to a light guide (not shown) which extends from the light source connector 11a to a distal end portion of the endoscope 2, and the illumination light is radiated from the distal end portion of the endoscope 2. The electric connector 11b is connected to a video processor 14 via an electric cable 13. An image signal, which is captured by an image pick-up unit (not shown) at the distal end portion of the endoscope 2, is output to the video processor 14 via a signal cable, which extends from the distal end portion of the endoscope 2 to the electric connector 11b, and the electric cable 13. The video processor 14 processes the input image signal, and causes a monitor to display an observation image. In the meantime, the operation section 8 of the endoscope 2 is provided with various switches 16 for operating the video processor 14.

The overtube 3 includes a tubular member 21 which constitutes the main body of the overtube 3. The insertion section 4 of the endoscope 2 is advancibly/retreatably inserted into the inner cavity of the tubular member 21 from a proximal end opening to a distal end opening. A distal-end cap 22 is provided at a distal end portion of the tubular member 21.

A water-feed mouthpiece 23 and an air-feed/discharge mouthpiece 24 are provided at a proximal end portion of the tubular member 21. The water-feed mouthpiece 23 communicates with the inner cavity of the tubular member 21 via a liquid supply conduit 25 which serves as a liquid transfer path. A lubricant can be supplied into the inner cavity of the tubular member 21 from the water-feed mouthpiece 23 via the liquid supply conduit 25, for example, by a syringe (not shown) which functions as a liquid transfer device.

A balloon 26 is fitted on a distal end portion of the tubular member 21. A gas feed/discharge conduit 27, which serves as a gas transfer path and couples the air-feed/discharge mouthpiece 24 and balloon 26, is provided on a peripheral wall portion of the tubular member 21. The balloon 26 can be inflated/deflated by feeding/discharging air from/to the air-feed/discharge mouthpiece 24 via the gas feed/discharge conduit 27.

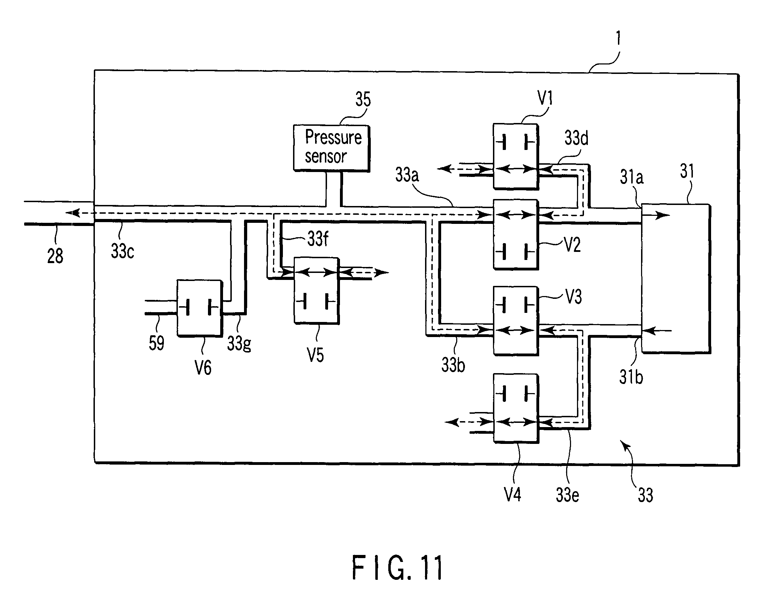

The balloon control apparatus 1 is connected to the air-feed/discharge mouthpiece 24 via a feed/discharge tube 28 (fluid feed/discharge conduit) 28 serving as a gas tube. The balloon control apparatus 1 includes a fluid circuit 33 (shown in FIG. 11), which includes a pump 31 (pressure source) for feeding/discharging a gas to/from the feed/discharge tube 28, and a control unit 32 which controls a suction operation from an opening end via the feed/discharge tube 28 on the basis of a pressure difference which is produced by the pump 31.

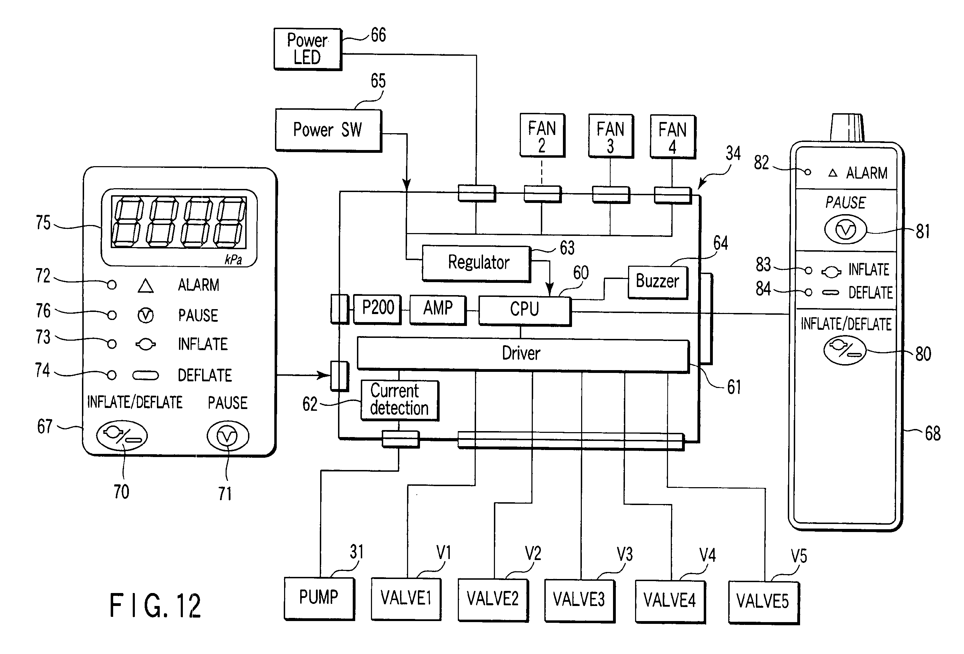

The control unit 32 includes a fluid circuit 33 for balloon control, which is built in the balloon control apparatus 1, and a board 34 (shown in FIG. 12) which executes control of respective structural components assembled in the fluid circuit 33.

FIG. 11 shows the fluid circuit 33 for controlling the balloon 26, which is built in the balloon control apparatus 1. The fluid circuit 33 includes the above-mentioned one pump 31, five (first to fifth) pinch valves V1 to V5, one relief valve V6, and one pressure sensor 35.

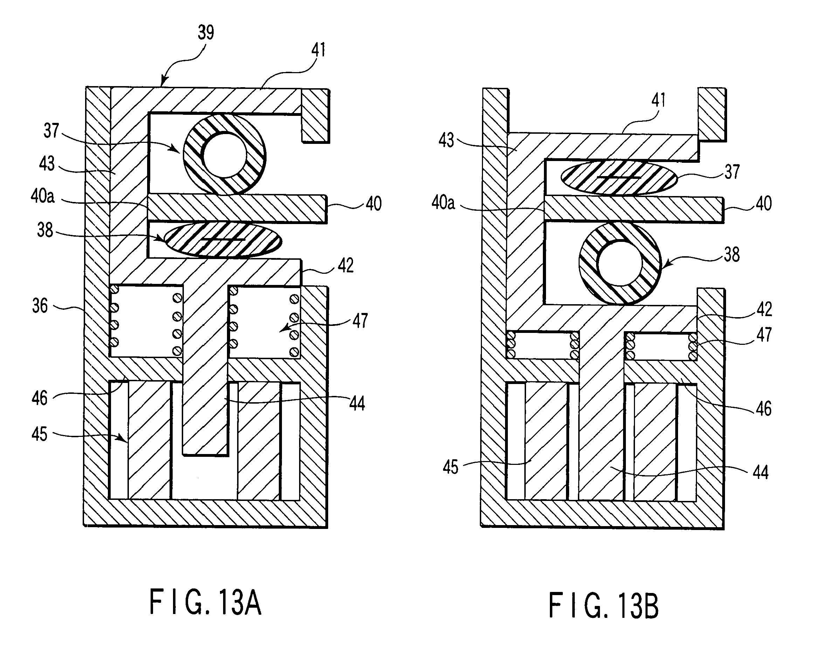

The first to fifth pinch valves V1 to V5 have the same structure. FIG. 13A and FIG. 13B schematically show the structure of one pinch valve V1. The pinch valve V1 includes one stationary frame 36, two (first and second) tubes 37 and 38, and a tube opening/closing member 39. A tube crush up member 40, which extends in a horizontal direction in FIG. 13A and FIG. 13B, is provided at an upper part of the stationary frame 36. The first tube 37 is provided on the upper side of the tube crush up member 40, and the second tube 38 is provided on the lower side of the tube crush up member 40.

The tube opening/closing member 39 includes an upper-side push member 41, a lower-side push member 42, a coupling member 43 and a slide member 44. The upper-side push member 41 is disposed on the upper side of the first tube 37, and the lower-side push member 42 is disposed on the lower side of the second tube 38. The coupling member 43 extends in a substantially vertical direction. One end portion of the upper-side push member 41 is fixed to an upper end portion of the coupling member 43. One end portion of the lower-side push member 42 is fixed to a lower end portion of the coupling member 43.

A hole portion 40a, in which the coupling member 43 is vertically movably inserted, is formed in the tube crush up member 40 of the stationary frame 36. The first tube 37 is detachably inserted between the upper-side push member 41 and the tube crush up member 40, and the second tube 38 is detachably inserted between the lower-side push member 42 and the tube crush up member 40.

The slide member 44 is downwardly projecting provided on a central part of the lower surface of the lower-side push member 42. A cylindrical solenoid (electromagnet) 45 is provided in a lower part of the stationary frame 36. The slide member 44 is vertically movably inserted in the solenoid 45.

A spring receiving member 46 is provided on the upper side of the solenoid 45. A spring member 47, such as a coil spring, is assembled between the spring receiving member 46 and the lower-side push member 42. The spring member 47 upwardly urges the tube opening/closing member 39.

The distance between the upper-side push member 41 and the lower-side push member 42 is set at such a dimension that one of the first and second tubes 37 and 38 is held in the open state and the other is held in the closed state, as shown in FIG. 13A or FIG. 13B. In the state in which no power is supplied to the solenoid 45 (power-off time), the tube opening/closing member 39 is held in the upwardly shifted position, as shown in FIG. 13A. At this time, the first tube 37 is held in the open state, and the second tube 38 is held in the shut-off (closed) state. In the state in which power is supplied to the solenoid 45, the tube opening/closing member 39 is switched to the downwardly shifted position against the spring force of the spring member 47, as shown in FIG. 13B. At this time, the first tube 37 is switched to the shut-off (closed) state, and the second tube 38 is switched to the open state. In the first to fifth pinch valves V1 to V5, the channel of the second tube 38, which is held in the closed state at the power-off time, is not used, and a dummy tube is disposed as the second tube 38.

As is shown in FIG. 11, the fluid circuit 33 includes an air suction conduit 33a, an air feed conduit 33b and an air feed/discharge conduit 33c. The pump 31 has a suction port 31a and a discharge port 31b. A proximal end portion of the suction conduit 33a is connected to the suction port 31a of the pump 31. A proximal end portion of the air feed conduit 33b is connected to the discharge port 31b of the pump 31. A distal end portion of the suction conduit 33a and a distal end portion of the air feed conduit 33b are connected to a proximal end portion of the air feed/discharge conduit 33c. A distal end portion of the air feed/discharge conduit 33c is connected to a proximal end portion of the feed/discharge tube 28.

The second pinch valve V2 is provided on the air suction conduit 33a. The third pinch valve V3 is provided on the air feed conduit 33b. One end of a branch conduit 33d for introducing outside air is connected to the air suction conduit 33a between the second pinch valve V2 and the suction port 31a of the pump 31. The first pinch valve V1 is provided on the branch conduit 33d.

One end of a branch conduit 33e for outside discharge is connected to the air feed conduit 33b between the third pinch valve V3 and the discharge port 31b of the pump 31. The fourth pinch valve V4 is provided on the branch conduit 33e.

The pressure sensor 35 is connected to the air feed/discharge conduit 33c. One end of each of two branch conduits 33f and 33g is connected to the air feed/discharge conduit 33c. The fifth pinch valve V5 is provided on one of the branch conduits, 33f. The relief valve V6 is provided on the other branch conduit 33g.

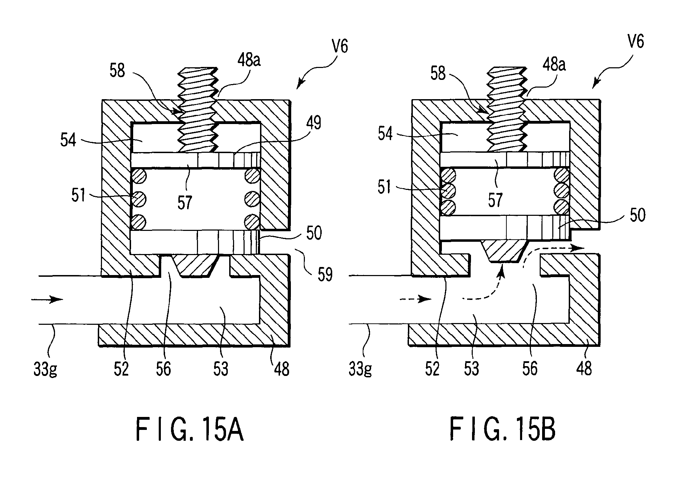

FIG. 15A and FIG. 15B schematically show the structure of the relief valve V6. The relief valve V6 includes a valve case 48, a valve support member 49, a valve body 50 and a coil spring 51. The inside of the valve case 48 is divided into two chambers (first chamber 53 and second chamber 54) by a partition wall 52 which functions also as a valve seat. The first chamber 53 is connected to the branch conduit 33g of the fluid circuit 33 within the balloon control apparatus 1. The valve support member 49, valve body 50 and coil spring 51 are contained in the second chamber 54. An opening portion 56 is formed in a central part of the partition wall 52. The valve body 50 is disposed in the state in which the valve body 50 openably closes the opening portion 56 of the partition wall 52.

The valve support member 49 includes a spring receiving portion 57 and a male screw portion 58. The male screw portion 58 is projectingly provided on a central part of the spring receiving portion 57 so as to project in a direction opposite to the valve body 50. The male screw portion 58 is engagingly inserted in a screw hole 48a which is formed in the valve case 48. The coil spring 51 is disposed between the valve support member 49 and the valve body 50. Normally, the valve body 50 is held in the state in which the valve body 50 is urged by the spring force of the coil spring 51 in such a direction as to close the opening portion 56 of the partition wall 52. In the relief valve V6 of the present embodiment, the opening pressure, by which the valve body 50 is opened, is set at a proper pressure value, for example, 10.8 kPa.

A leak hole 59, which is opened/closed by the valve body 50, is formed in that area of the peripheral wall of the valve case 48, which is located on the second chamber 54 side. Until the pressure in the branch conduit 33g of the fluid circuit 33 reaches a preset pressure value, the relief valve V6 is held in the closed state, as shown in FIG. 15A, in which the valve body 50 closes the opening portion 56 of the partition wall 52. If the pressure in the branch conduit 33g of the fluid circuit 33 reaches the preset pressure value, the valve body 50 of the relief valve V6 is urged in a direction away from the partition wall 52, as shown in FIG. 15B, and the opening portion 56 is opened. At this time, as indicated by a broken-line arrow in FIG. 15B, the air, which comes in the first chamber 53 of the valve case 48 from the branch conduit 33g, is let to leak to the outside from the opening portion 56 via the leak hole 59.

FIG. 12 shows the control board 34 of the respective structural devices that are assembled in the fluid circuit 33. The control board 34 includes a CPU 60, a driver circuit 61, a current detection circuit 62, a regulator 63 and a buzzer 64. The first to fifth pinch valves V1 to V5 are connected to the CPU 60 via the driver circuit 61. The pump 31 is connected to the CPU 60 via the current detection circuit 62 and driver circuit 61. The regulator 63 and buzzer 64 are connected to the CPU 60. A power switch 65 (to be described later) and a display LED 66, which is lighted when the power switch 65 is turned on, are connected to the CPU 60 via the regulator 63. Further, an operation panel (instruction means) 67, which is a first controller, and a remote controller (instruction means) 68, which is a second controller, are connected to the control board 34.

The operation panel 67 is fixed to a front panel 69a of a box-shaped outer case 69 of the balloon control apparatus 1. The remote controller 68 is connected to the balloon control apparatus 1 via a connection cord.

The operation panel 67 includes an inflate/deflate switch 70, a pause switch 71, an alarm display lamp 72, an inflation display lamp 73, a deflation display lamp 74, a pause display lamp 76, and a pressure display panel 75. The remote controller 68 includes an inflate/deflate switch 80, a pause switch 81, an alarm display lamp 82, an inflation display lamp 83 and a deflation display lamp 84.

The balloon control apparatus 1 of the present embodiment is controlled on the basis of the operation of the power switch 65, and the operation of the inflate/deflate switch 70 and pause switch 71 of the operation panel 67 or the operation of the inflate/deflate switch 80 and pause switch 81 of the remote controller 68.

When the power switch 65 is operated and the inflate/deflate switch 70 of the operation panel 67 or the inflate/deflate switch 80 of the remote controller 68 is operated, the structural devices that are assembled in the fluid circuit 33 operate as shown in Table 1.

**Table 1**

| | V1 | V2 | V3 | V4 | V5 | PUMP | V6 |
|---|---|---|---|---|---|---|---|
| Power-off | Open | Open | Open | Open | Open | OFF | Closed |
| Power-on | Open | Open | Open | Open | Open | OFF | Closed |
| Conduit hold | Open | Closed | Closed | Open | Closed | OFF | Closed |
| Air feed (balloon inflation) | Open | Closed | Open | Closed | Closed | ON | Closed |
| Suction (balloon deflation) | Closed | Open | Closed | Open | Closed | ON | Closed |
| Pressure sensor abnormality (at time of display "over") | - | - | - | - | - | - | Open |
| Electromagnetic valve Open abnormality (mechanical failure) | Open | Open | Open | Open | Open | OFF | Closed |

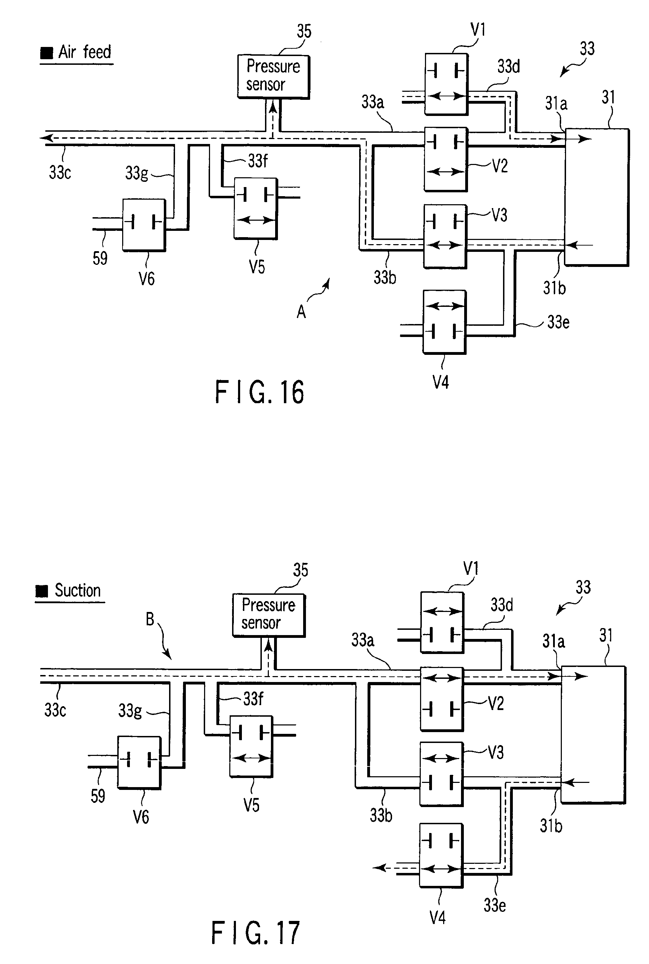

Thereby, when air is fed to the balloon 26, a flow path A for air feed is formed, as indicated by broken lines in FIG. 16. In the flow path A, outside air is sucked from the valve V1 via the branch conduit 33d for introducing outside air, by the suction function of the suction port 31a of the pump 31. At the same time, air, which is discharged from the discharge port 31b of the pump 31, is fed to the air feed/discharge conduit 33c from the air feed conduit 33b via the third pinch valve V3. At this time, the pressure in the air feed/discharge conduit 33c is detected by the pressure sensor 35.

When air is sucked from the balloon 26, a flow path B for suction is formed as indicated by broken lines in FIG. 17. In the flow path B, a suction pressure acts in the air feed/discharge conduit 33c via the second pinch valve V2 of the air suction conduit 33a by the suction function of the suction port 31a of the pump 31. At the same time, air, which is discharged from the discharge port 31b of the pump 31, flows from the air feed conduit 33b to the branch conduit 33e side for outside discharge, and the air is discharged to the outside via the fourth pinch valve V4. At this time, the pressure in the air feed/discharge conduit 33c is detected by the pressure sensor 35.

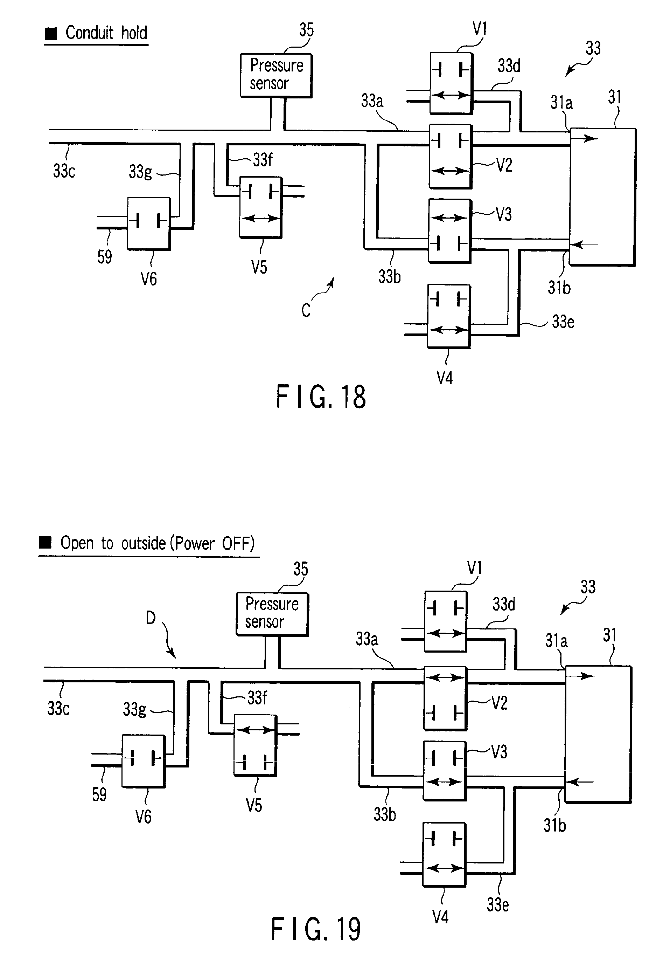

When the conduits are in the hold state, a flow path C is formed as show in FIG. 18. At this time, the pressure in the air feed/discharge conduit 33c is detected by the pressure sensor 35. At the time of opening to outside (power off), a flow path D shown in FIG. 19 is formed.

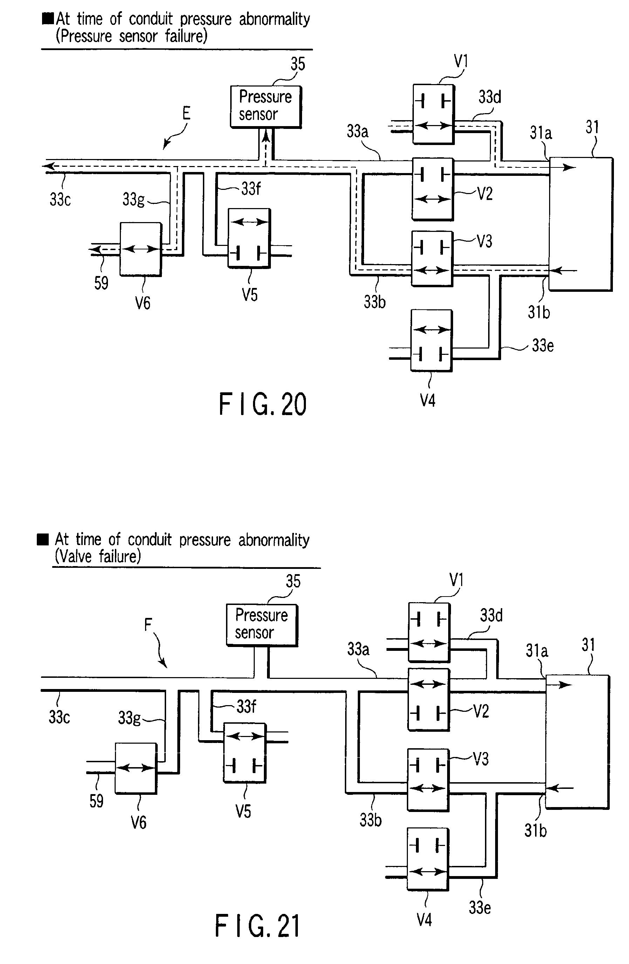

At a time of conduit pressure abnormality due to a failure of the pressure sensor 35, a flow path E shown in FIG. 20 is formed. At a time of conduit pressure abnormality due to a valve failure, a flow path F shown in FIG. 21 is formed. In each of the flow paths E and F, the relief valve V6 operates.

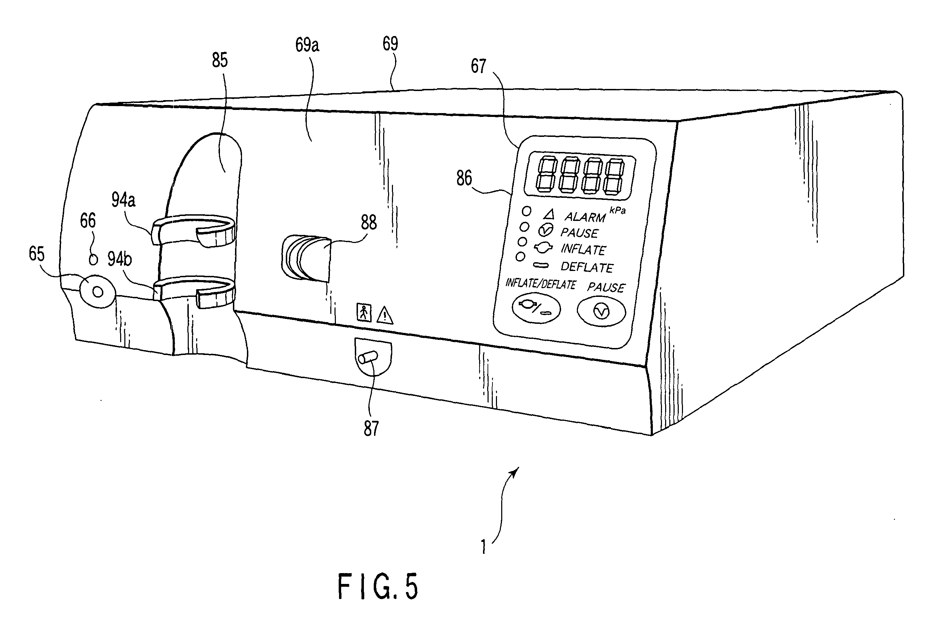

FIG. 5 shows the external appearance of the balloon control apparatus 1 according to the present embodiment. The main body of the balloon control apparatus 1 has the box-shaped outer case 69. The front panel 69a of the case 69 has the above-described power switch 65, a concave-shaped bottle receiving recess portion 85, a mount portion 86 of the operation panel 67, an air feed/discharge port portion 87 which is a pressure source side led-out portion of the air feed/discharge conduit 33c, and a tube holder 88.

Further, the balloon control apparatus 1 includes a bottle (reservoir means) 89 for liquid recovery, which recovers a liquid, such as a body fluid, in a case where the balloon 26 is broken and a body fluid reversely flows into the feed/discharge tube 28. The bottle 89 communicates with the feed/discharge tube 28, and has a preset predetermined capacity for storing a liquid. The capacity of the bottle 89 is determined by a maximum capacity of a liquid which is sucked, for example, in a predetermined time (time-out period = 20 seconds), in combination with the feed/discharge tube 28 that is connected. In accordance with the suction operation based on the control by the control unit 32, the liquid that moves in the feed/discharge tube 28 is recovered.

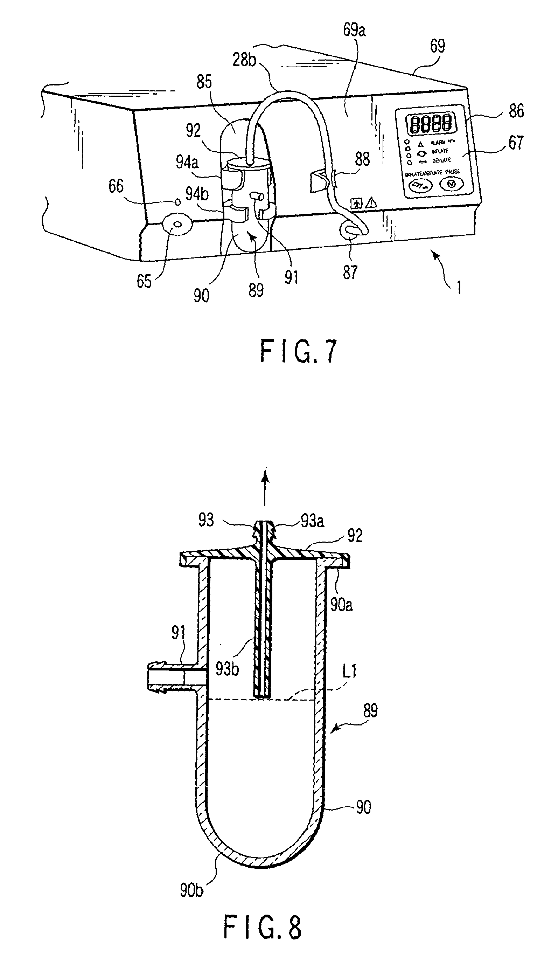

As shown in FIG. 8, the bottle 89 has a bottomed cylindrical bottle body 90. The bottle body 90 is formed of, for example, a transparent resin material. An upper surface part of the bottle body 90 is opened. An annular flange portion 90a, which is bent outward, is formed at an outer peripheral edge of the upper opening portion of the bottle body 90. A first connection mouthpiece 91 is projectingly provided on an outer peripheral surface of the bottle body 90. The first connection mouthpiece 91 is disposed at a substantially middle position in the vertical direction of the bottle body 90. A substantially hemispherical surface 90b is formed at the bottom of the bottle body 90.

The upper opening portion of the bottle body 90 is closed by a disc-shaped cover 92. A second connection mouthpiece 93 is projectingly provided on a substantially central part of the cover 92. An upper end portion 93a of the second connection mouthpiece 93 projects in an upward direction of the cover 92. A lower end portion 93b of the second connection mouthpiece 93 projects inward of the bottle body 90. A projection end of the lower end portion 93b of the second connection mouthpiece 93 extends lower than a lower end portion of the first connection mouthpiece 91.

The bottle 89 is configured by such presetting that a liquid level L1 at a time when the amount of recovered liquid has reached a predetermined capacity level (full liquid level) becomes lower than the projection end of the lower end portion 93b of the second connection mouthpiece 93, as indicated by a broken line in FIG. 8. Specifically, in FIG. 8, the lower-side position of the projection end portion of the lower end portion 93b of the second connection mouthpiece 93 is set at the position of the maximum liquid level L1, up to which the bottle 89 can store the liquid.

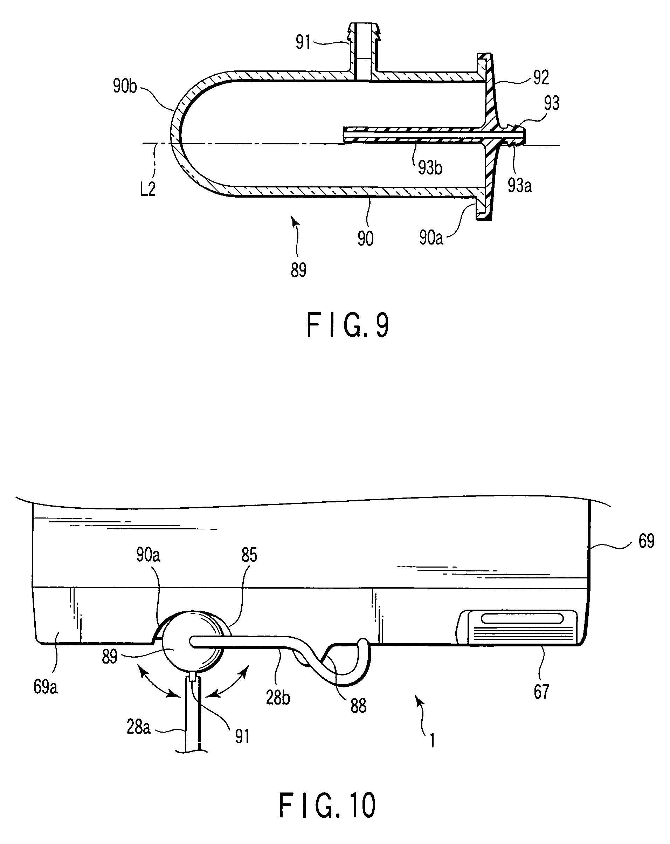

Further, as shown in FIG. 9, the bottle 89 of the present embodiment is configured by such presetting that even in the case where the bottle 89 is horizontally fallen, a liquid level L2 at a time when the amount of recovered liquid has reached a predetermined capacity level (full liquid level) becomes lower than the projection end of the lower end portion 93b of the second connection mouthpiece 93, as indicated by a broken line in FIG. 9. Thereby, even in the case where the bottle 89 is horizontally fallen, the liquid recovered in the bottle 89 is prevented from leaking. Moreover, even in the case where the bottle 89 is turned upside down, the liquid recovered in the bottle 89 is prevented from leaking.

The bottle 89 is received in the bottle receiving recess portion 85 of the front panel 69a of the balloon control apparatus 1. As shown in FIG. 5, a pair of upper and lower C-shaped bottle holders (bottle holding portions) 94a and 94b are provided on the bottle receiving recess portion 85 of the case 69. The inside diameter of the C-shaped bottle holder 94a, 94b is set to be substantially equal to the outside diameter of the bottle 89. The bottle 89 is inserted in the C-shaped bottle holder 94a, 94b. In the state in which the flange portion 90a of the bottle body 90 abuts on the upper end portion of the upper-side C-shaped bottle holder 94a, the bottle 89 is detachably mounted in the pair of upper and lower C-shaped bottle holders 94a and 94b. Thereby, when the bottle 89 is mounted in the C-shaped bottle holders 94a and 94b, the bottle 89 is held by the upper and lower C-shaped bottle holders 94a and 94b so as to be rotatable about the center axis of the bottle 89 in the state in which the center axis of the bottle body 90 is set in the horizontal direction.

The feed/discharge tube 28 includes a medical-equipment-side conduit 28a which is connected to the balloon 26 side, and a pressure-source-side conduit 28b which is connected to the air feed/discharge conduit 33c within the balloon control apparatus 1. A distal end portion of the medical-equipment-side conduit 28a is connected to the air-feed/discharge mouthpiece 24 of the overtube 3. A proximal end portion of the medical-equipment-side conduit 28a is connected to the first connection mouthpiece 91 of the bottle 89. A distal end portion of the pressure-source-side conduit 28b is connected to the upper end portion 93a of the second connection mouthpiece 93 of the bottle 89. A proximal end portion of the pressure-source-side conduit 28b is connected to the air feed/discharge port portion 87 of the balloon control apparatus 1. The pressure-source-side conduit 28b between the second connection mouthpiece 93 of the bottle 89 and the air feed/discharge port portion 87 of the balloon control apparatus 1 is held by the tube holder 88.

In the present embodiment, operation-canceling means for stopping the operation of suction of the liquid into the bottle 89 is provided in order to limit the amount of recovered liquid in the bottle 89 within a predetermined capacity. This operation-canceling means includes at least one of means for stopping the pump 31 after passing of a preset predetermined time (e.g. 20 seconds), means (fifth pinch valve V5) for shutting the air feed/discharge conduit 33c within the balloon control apparatus 1 after the passing of the preset time, and means (relief valve V6) for leak to the outside of the air feed/discharge conduit 33c within the balloon control apparatus 1 after the passing of the preset time.

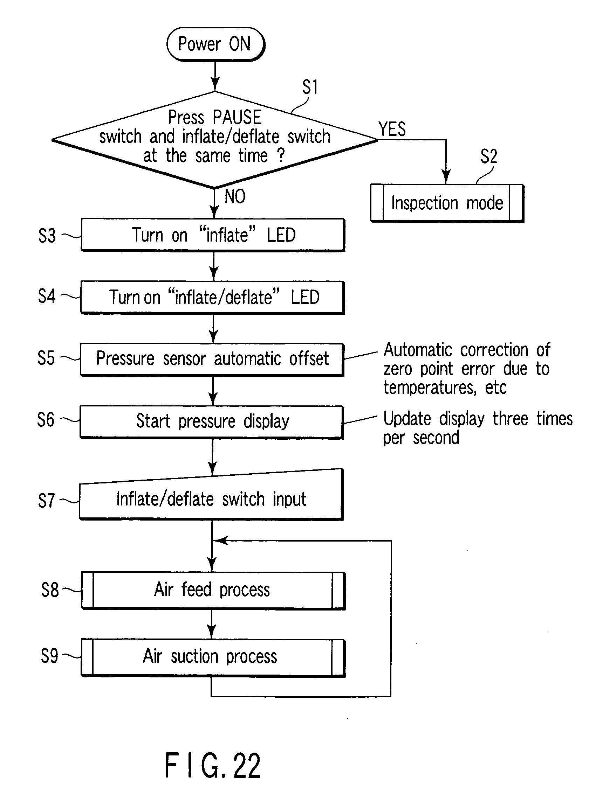

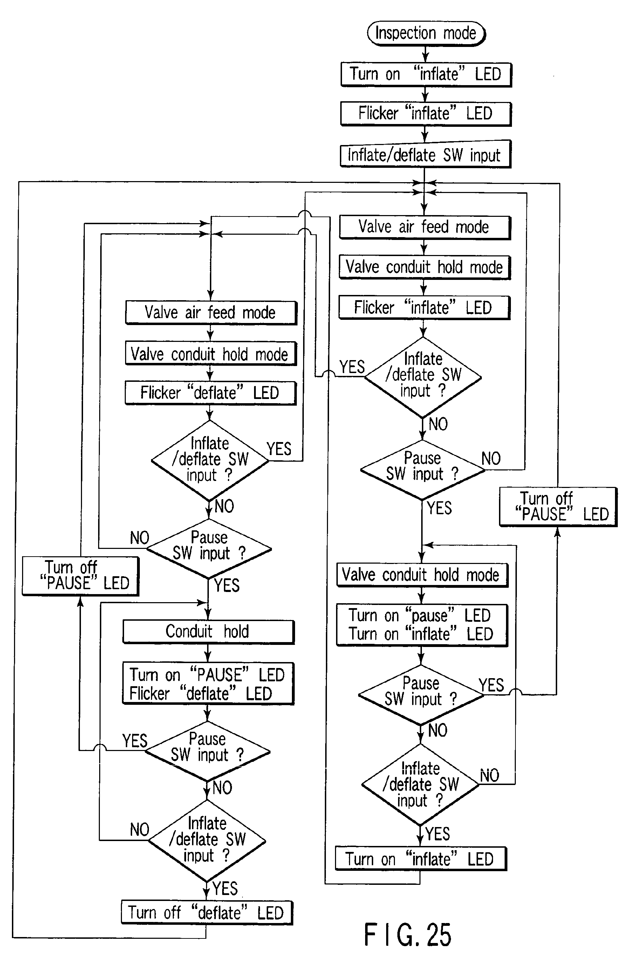

Next, the operation of the above-described structure is described. The balloon control apparatus 1 that is the medical apparatus of the present embodiment is used in combination with the endoscope system shown in FIG. 1. FIG. 22 is a flow chart for describing the main operation of the of the balloon control apparatus 1. If the power switch 65 is turned on, a first step S1 is executed. In step S1, it is determined whether the pause switch 71 and the inflate/deflate switch 70 are pressed at the same time in a simultaneous switch-on state. If the simultaneous switch-on state is determined, the control advances to an inspection mode (see FIG. 25) (step S2). If the simultaneous switch-on state is not determined in step S1, the control advances to the next step S3, and the inflation display lamp 73 is lighted. Subsequently, the inflation/deflation LED is lighted in step S4, and automatic offset of the pressure sensor 35 is executed in step S5. In this case, automatic correction of a zero point error due to temperatures is executed. In the next step S6, pressure display on the pressure display panel 75 is started. In step S7, the inflate/deflate switch 70 is input, and successively an air feed process is executed in step S8 and an air suction process is executed in step S9.

When the endoscope 2 is to be inserted into a body cavity, the insertion section 4 of the endoscope 2 is inserted in advance into the overtube 3, as indicated by an arrow in FIG. 2, and the overtube 3 and the insertion section 4 of the endoscope 2 are integrally fixed. At this time, sterile water is injected into the inner cavity of the tubular member 21 of the overtube 3 from the water-feed mouthpiece 23 via the liquid supply conduit 25 by means of, e.g. a syringe. Thereby, the inner surface of the insertion section 4 of the overtube 3 is wetted with the sterile water. Excess sterile water is drained.

Then, the insertion section 4 of the endoscope 2 is inserted from the proximal end opening (endoscope insertion hole) of the tubular member 21 of the overtube 3, and the insertion section 4 is passed through the inner cavity of the tubular member 21. The bending section 6 of the endoscope 2 is projected from the distal end of the overtube 3, and the distal end of the overtube 3 is positioned in front of the bending section 6 of the endoscope 2. At this time, the endoscope insertion hole of the overtube 3 and the position of the insertion section 4 of the endoscope 2 are confirmed. This position serves as an approximate reference for the limit of insertion of the overtube 3 along the endoscope 2.

Subsequently, the air-feed/discharge mouthpiece 24 of the overtube 3 and the air feed/discharge port portion 87 of the balloon control apparatus 1 are connected via the feed/discharge tube 28. The bottle 89 for liquid recovery is provided on the feed/discharge tube 28.

In this state, the overtube 3 and the endoscope 2 are inserted as one piece into the body cavity. When the overtube 3 and endoscope 2 have reached a position in front of a bent part of the body cavity, the endoscope 2 is retreated relative to the overtube 3.

Subsequently, a gas is fed into the balloon 26 from the air-feed/discharge mouthpiece 24 via the gas feed/discharge conduit 27 by the balloon control apparatus 1, and the balloon 26 is inflated and engaged with the inner surface of the body wall. In this state, the overtube 3 is retreated, and the body wall is pulled to the near side. Thereby, the bent body cavity is straightened. Then, the endoscope 2 is advanced relative to the overtube 3, and is made to pass through the bent part of the body cavity.

At the time when the endoscope 2 is advanced to a position in front of the next bent part of the body cavity, the gas is discharged from the balloon 26 of the overtube 3 by the balloon control apparatus 1. Thereby, the balloon 26 is deflated and the engagement between the balloon 26 and the inner surface of the body wall is released. Subsequently, the overtube 3 is advanced along the endoscope 2 to the position in front of the bent part, and the balloon 26 is inflated once again and engaged with the inner surface of the body wall. In this case, up to the position where the overtube 3 is inserted, the body cavity is held in such a shape as to allow easy insertion. Subsequently, similar operations are repeated, and the endoscope 2 is inserted into a deeper part of the body cavity which is bent in a complex shape. When necessary, the overtube 3 and the endoscope 2 are fixed and unfixed.

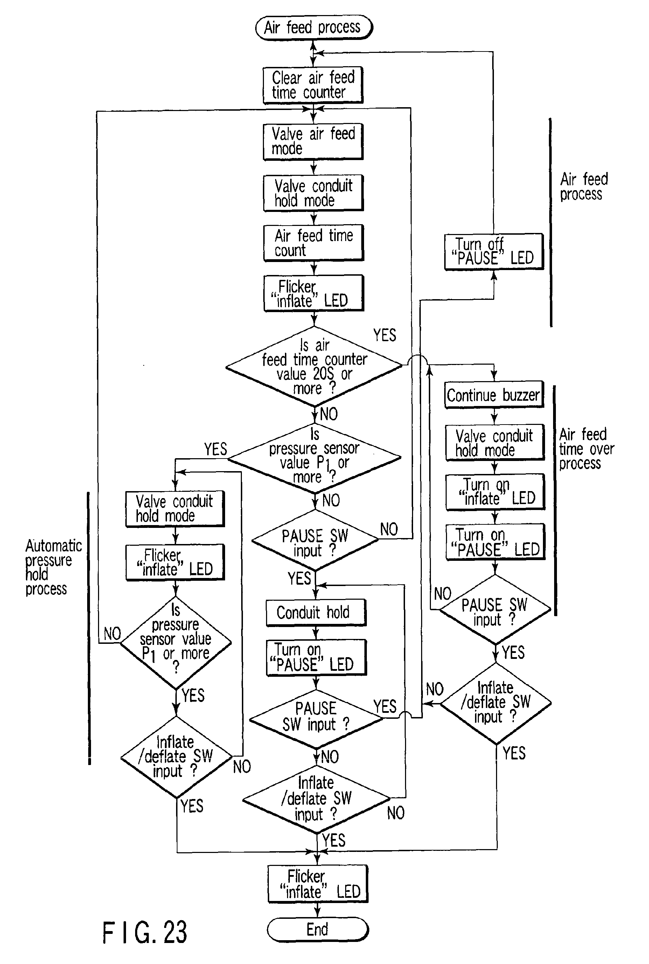

When the air feed process (balloon inflation) is executed by the balloon control apparatus 1, the operation illustrated in a flow chart of FIG. 23 is performed. Specifically, when the balloon 26 is inflated, the following operation is performed.
1. The inflate/deflate switch 70 of the operation panel 67 or the inflate/deflate switch 80 of the remote controller 68 is pressed. Thereby, air feed to the balloon 26 is started. When air is fed to the balloon 26, the flow path A at the time of air feed, as indicated by the broken lines in FIG. 16, is formed in the fluid circuit 33 within the balloon control apparatus 1. Thereby, the air feed to the balloon 26 is executed. While the balloon 26 is being inflated, the inflation display lamp 73, 83 is flickered.
2. If the pressure in the balloon 26 reaches an upper-limit set pressure P1 (e.g. P1 = 5 kPa) at the time of air feed, the air feed is stopped and the balloon pressure is maintained. At this time, the inflation display lamp 73, 83 is lighted.

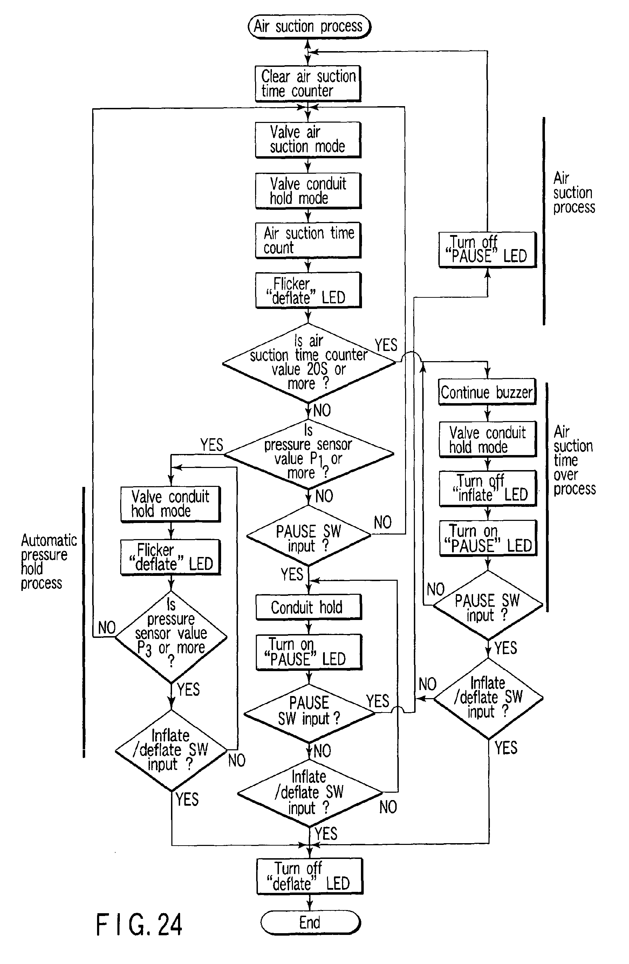

When the air suction process (balloon deflation) is executed by the balloon control apparatus 1, the operation illustrated in a flow chart of FIG. 24 is performed. Specifically, when the balloon 26 is deflated, the following operation is performed.
1. When the balloon 26 is deflated, the inflate/deflate switch 70 of the operation panel 67 or the inflate/deflate switch 80 of the remote controller 68 is pressed once again. Thereby, air suction from the balloon 26 is started. At this time, the flow path B at the time of air suction, as indicated by the broken lines in FIG. 17, is formed in the fluid circuit 33 within the balloon control apparatus 1. Thereby, the air suction from the balloon 26 is executed. While the balloon 26 is being deflated, the deflation display lamp 74, 84 is flickered.
2. If the pressure in the balloon 26 reaches a lower-limit set pressure P3 (e.g. P3 = -6 kPa) or less at the time of air suction, the air suction is stopped and the balloon pressure is maintained. At this time, the deflation display lamp 74, 84 is lighted.

In the case where the state of the balloon 26 is to be held while the balloon 26 is being inflated or deflated, the pause switch 71 of the operation panel 67 or the pause switch 81 of the remote controller 68 is pressed. At this conduit hold time, the flow path C shown in FIG. 18 is formed. During the pause, the pause display lamp 76 is lighted. When the inflation or deflation is to be resumed, the pause switch 71 or 81 is pressed once again.

If the inflate/deflate switch 70, 80 is pressed while the balloon 26 is being inflated, the balloon 26 is deflated. Further, if the inflate/deflate switch 70, 80 is pressed while the balloon 26 is being deflated, the balloon 26 is inflated.

In the case where air feed is performed for 20 seconds or more after the inflation of the balloon 26 is started, the following operation is performed. Specifically, if the cumulative time of air feed has become 20 seconds since the start of inflation of the balloon 26, an alarm sound is produced intermittently and the state of the balloon 26 is held. In addition, the alarm display lamp 72, 82 is flickered. In the meantime, if a pause is executed while the balloon 26 is being inflated, the time is re-measured from the beginning of re-start of inflation.

If the state, in which the pressure of the balloon 26 does not lower to the lower-limit set pressure P3 (e.g. P3 = -6 kPa) or less from the beginning of deflation of the balloon 26, continues for 20 seconds or more, the following operation is performed. Specifically, the inflate/deflate switch 70, 80 is pressed, and the state, in which the pressure of the balloon 26 does not lower to the lower-limit set pressure P3 or less from the beginning of deflation of the balloon 26, continues for 20 seconds or more, an alarm sound is produced intermittently and the state of the balloon 26 is held. If a pause is executed while the balloon 26 is being deflated, the time is re-measured from the beginning of re-start of deflation.

If the pressure in the air feed/discharge conduit 33c within the balloon control apparatus 1 rises to P2 (e.g. P2 = 8 kPa) or more while the balloon control apparatus 1 is being operated, a continuous alarm is produced. Moreover, if this state continues for five seconds, occurrence of abnormality is determined and the alarm sound is changed to an intermittent alarm sound, and the balloon conduits are opened. At this time of opening to outside (power off), the flow path D shown in FIG. 19 is formed. In the case where the balloon 26 is broken during the suction operation of the balloon control apparatus 1 and a body fluid reversely flows into the feed/discharge tube 28, the body fluid is recovered in the bottle 89 for liquid recovery. At this time, the pump 31 is stopped by the operation-canceling means after passing of a preset time (e.g. 20 seconds), and the suction operation of sucking the body fluid into the bottle 89 is canceled. Thereby, the amount of recovered liquid in the bottle 89 is limited within the predetermined capacity.

The following advantageous effects can be obtained by the above-described structure. Specifically, in the present embodiment, in the case where the balloon 26 is broken during the suction operation of the balloon control apparatus 1 and a body fluid reversely flows into the feed/discharge tube 28, the pump 31 is stopped by the operation-canceling means after passing of a preset time (e.g. 20 seconds), and the suction operation of sucking the body fluid into the bottle 89 is canceled. Thereby, the amount of recovered liquid in the bottle 89 is limited to the predetermined capacity. Therefore, even if the body fluid reversely flows into the feed/discharge tube 28, no body fluid overflows from the bottle 89, and the liquid can properly be recovered into the bottle 89. Moreover, in the present embodiment, there is no need to provide special sensing means, such as a liquid level sensor, in the vicinity of the suction bottle, and an increase in manufacturing cost can be suppressed.

In the present embodiment, when the bottle 89 is mounted in the C-shaped bottle holders 94a and 94b, the bottle 89 is held by the upper and lower C-shaped bottle holders 94a and 94b so as to be rotatable about the center axis of the bottle 89 in the state in which the center axis of the bottle body 90 is set in the horizontal direction. Thereby, as shown in FIG. 10, the bottle 89, which is held by the C-shaped bottle holders 94a and 94b, can be rotated, while in use, about the center axis of the bottle 89, and the direction of the first connection mouthpiece 91 of the bottle 89 can freely be changed. Therefore, advantageously, the medical-equipment-side conduit 28a does not become an obstacle.

In addition, in the present embodiment, the fluid circuit 33 of the balloon control apparatus 1 includes at least the air feed flow path A which causes the discharge pressure of the pump 31 to act on the bottle 89 side, and the suction flow path B which causes the suction pressure of the pump 31 to act on the bottle 89 side. The air feed flow path A and suction flow path B are switched by the control unit 32 in accordance with an instruction from the inflate/deflate switch 70 of the operation panel 67 or the inflate/deflate switch 80 of the remote controller 68. Thereby, the single pump 31 can be shared at the time of air feed and at the time of air suction. Therefore, the number of pumps 31, which are assembled in the fluid circuit 33 of the balloon control apparatus 1, can be decreased, and the manufacturing cost can be reduced.

In the present embodiment, since the pump 31 is operated in accordance with the instruction from the inflate/deflate switch 70 of the operation panel 67 or the inflate/deflate switch 80 of the remote controller 68, the pump 31 can be operated only when necessary. Therefore, the operation of the pump 31 can be suppressed, the power consumption can be reduced, and the sound that is produced can be decreased.

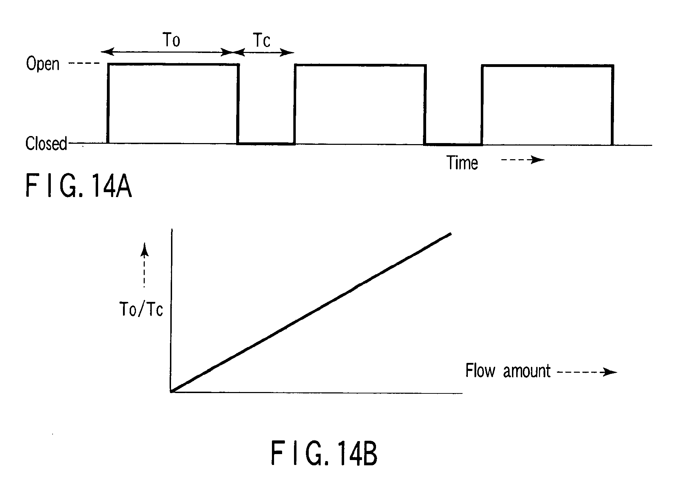

Furthermore, in the present embodiment, the air flow amount at the time of air feed or air suction can be adjusted by controlling the opening/closing of the pinch valves. At the time of air feed, one or both of the third pinch valve V3 of the air feed conduit 33b and the first pinch valve V1 on the branch conduit 33d are used. At the time of air suction, one or both of the second pinch valve V2 of the air suction conduit 33a and the fourth pinch valve V4 on the branch conduit 33e are used. Specifically, by varying the ratio between an opening time To and a closing time Tc of each pinch valve as shown in FIG. 14A, the air flow amount can be adjusted as shown in FIG. 14B. Thereby, the flow amount at the time of air feed to the balloon 26 can be adjusted without adding dedicated components.

In each of the first to fifth pinch valves V1 to V5 of the present embodiment, the dummy tube is provided as the second tube 38 which is held in the closed state at the time of power-off. Consequently, in the case where the apparatus is not used for a long time, sticking of the dummy tube that is held in the closed state may occur at the time of power-off. Thereby, even in the case where the apparatus is not used for a long time, the "sticking" phenomenon of the first tube 37 that is actually used can be prevented.

In the present embodiment, the fifth pinch valve V5 is provided on the branch conduit 33f that is connected to the air feed/discharge conduit 33c. The fifth pinch valve V5 is set to be opened by a dedicated conduit opening valve CPU (software) when the conduit pressure varies to an abnormal level of about P2 (e.g. P2 = 8 kPa). Therefore, since the air feed/discharge conduit 33c can be opened by the fifth pinch valve V5 at the time of an abnormal conduit pressure of the air feed/discharge conduit 33c, the abnormality in conduit pressure can be avoided, and the safety of the balloon control apparatus 1 can be improved.

In the present embodiment, the relief valve V6, which is not subjected to electronic control and is opened by the conduit pressure, is provided on the branch conduit 33g that is coupled to the air feed/discharge conduit 33c. The opening pressure of the relief valve V6 is adjusted at P4 (e.g. P4 = 10 kPa). It is thus possible to avoid an abnormal conduit pressure state in which the conduit pressure of the air feed/discharge conduit 33c rises to P4 or more, and the safety of the balloon control apparatus 1 can further be improved.

The branch conduit 33f communicating with the fifth pinch valve V5 and the branch conduit 33g communicating with the relief valve V6 are located at positions closer to the air feed/discharge tube 28 than the other conduits of the fluid circuit. Hence, even in the case where abnormality occurs in other fluid circuits, the conduits including the balloon 26 are opened.

In the present embodiment, in the operation panel 67, the pressure display panel 75 is provided with the pressure display function, and the alarm display function is provided by the alarm display lamp 72. Thereby, excessive air feed at the time of air feed to the balloon 26 can be prevented, and the safety can further be improved.

Besides, the remote controller 68, which has the same functions as the operation panel 67 of the balloon control apparatus 1, is connected to the balloon control apparatus 1. Thus, the operation panel 67 and the remote controller 68 can selectively be used according to necessity. Therefore, the operability of the balloon control apparatus 1 can be improved. In addition, even at a time of a fault of the remote controller 68, the balloon control apparatus 1 can be operated by the operation panel 67 of the balloon control apparatus 1.

In the present embodiment, the balloon control apparatus 1 is configured to be controlled by the operation panel 67 and the remote controller 68. However, as shown in FIG. 1, a foot switch 100 may further be connected to the balloon control apparatus 1, and the balloon control apparatus 1 may be configured to be controlled by the foot switch 100. Besides, in the present embodiment, the pump 31 is configured to be continuously driven when the inflate/deflate switch 70 of the operation panel 67 and the inflate/deflate switch 80 of the remote controller 68 are operated. Alternatively, a mode change-over switch may additionally be provided, and the pump 31 may be configured to be driven by the mode change-over switch in such a manner that the pump 31 is driven only while the inflate/deflate switch 70 of the operation panel 67 and the inflate/deflate switch 80 of the remote controller 68 are actually being operated.

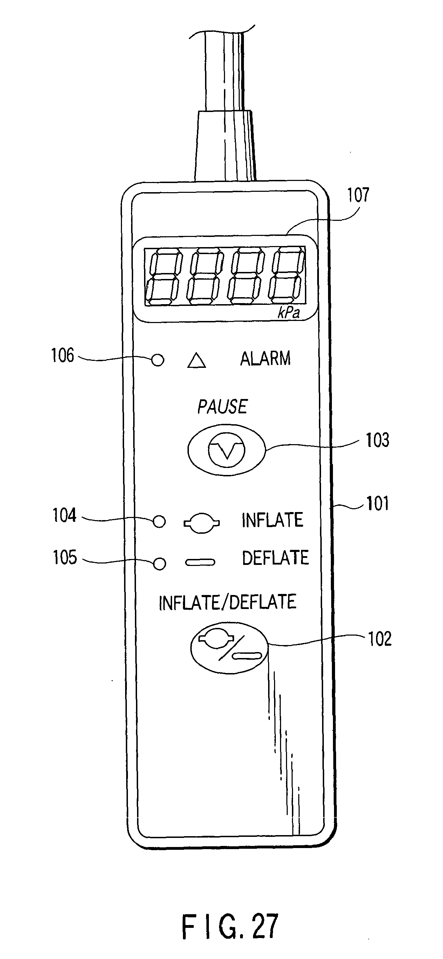

FIG. 27 shows a second embodiment of the present invention. In the present embodiment, the structure of the remote controller 68 of the first embodiment (see FIG. 1 to FIG. 26) is altered as follows.

Specifically, a remote controller 101 of the present embodiment includes an inflate/deflate switch 102, a pause switch 103, an inflation display lamp 104, a deflation display lamp 105, an alarm display lamp 106, and a pressure display panel 107.

In the present embodiment, by viewing the pressure display panel 107 of the remote controller 101, it is possible to confirm the pressure state of the air feed/discharge conduit 33c of the balloon control apparatus 1. Thus, even without particularly viewing the pressure display panel 75 of the operation panel 67 that is fixed to the front panel 69a of the balloon control apparatus 1, the pressure state of the air feed/discharge conduit 33c of the balloon control apparatus 1 can be confirmed. Therefore, the handling operability of the balloon control apparatus 1 can be enhanced.

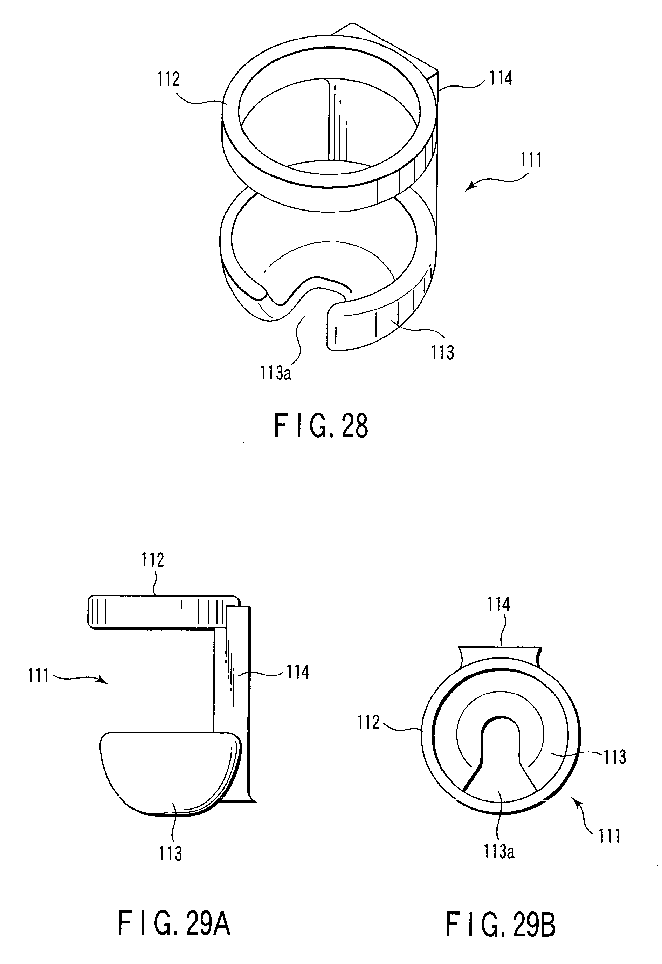

FIG. 28 and FIGS. 29A and 29B show a third embodiment of the present invention. In this embodiment, the structure of the bottle holder 94a, 94b, which is mounted in the bottle receiving recess portion 85 in the first embodiment (see FIG. 1 to FIG. 26), is altered as follows.

Specifically, a bottle holder 111 of the present embodiment includes an annular upper arm 112 which is disposed on the upper side, a substantially semispherical bottle receiving section 113 which is disposed on the lower side, and a flat-plate-shaped connection arm 114 which is vertically disposed. An upper end portion of the connection arm 114 is connected to one end portion of the upper arm 112. A lower end portion of the connection arm 114 is connected to one end portion of the bottle receiving section 113. A notch portion 113a is formed at a front end portion of the bottle receiving section 113. Thereby, when the bottle 89 is mounted in the bottle holder 111, the bottle 89 is held by the upper arm 112 and the bottle receiving section 113 so as to be rotatable about the center axis of the bottle 89 in the state in which the center axis of the bottle body 90 is set in the horizontal direction. Thus, the bottle 89, which is held by the bottle holder 111, can be rotated, while in use, about the center axis of the bottle 89, and the direction of the first connection mouthpiece 91 of the bottle 89 can freely be changed. Therefore, advantageously, the medical-equipment-side conduit 28a does not become an obstacle.

The first connection mouthpiece 91 of the bottle 89 is positioned between the upper arm 111 and the bottle receiving section 113. Thereby, even if the bottle 89 is pulled upward, the first connection mouthpiece 91 of the bottle 89 is hooked on the upper arm 112, and the bottle 89 is prevented from being removed from the bottle holder 111. In the meantime, although the balloon control apparatus 1 is provided with the single pump, one pump for inflating the balloon and another pump for deflating the balloon may be provided. In this case, in addition to the provision of the bottle 89 for the pump for suction, another bottle 89 may be provided on a conduit that is connected to the pump for air feed.

The present invention is not limited to the above-described embodiments. For example, in the above embodiments, the invention is applied to the balloon control apparatus 1. Alternatively, the invention may be applied to medical equipment other than the balloon 26, for instance, a suction apparatus which is connected to the suction conduit of the endoscope. Needless to say, various modifications may be made without departing from the spirit of the invention.

Next, other characteristic technical items of the present invention are described below.

### NOTE

(Item 1) A medical apparatus characterized by comprising medical equipment which is inserted in a body cavity; a pressure source which generates a pressure difference for suction via the medical equipment; a conduit which communicates with the pressure source and has an open end at the medical equipment; a control unit which controls suction via the conduit on the basis of the pressure difference that is generated by the pressure source, in accordance with an instruction from instruction means; reservoir means communicating with the conduit and having a predetermined capacity for recovering a liquid that moves in the conduit in accordance with the suction operation based on a control of the control unit; and operation-canceling means for canceling continuous suction, thereby to limit an amount of the recovered liquid within the predetermined capacity.

(Item 2) The medical apparatus according to item 1, characterized in that the medical equipment includes an insertion section which is inserted in the body cavity, the insertion section includes a balloon which inflates/deflates in accordance with the pressure difference, and the conduit permits communication between the pressure source and the balloon.

(Item 3) The medical apparatus according to item 1, characterized in that the operation-canceling means stops the pressure source, or shuts the conduit or prevents leak to outside of the conduit, after passing of a preset predetermined time.

(Item 4) The medical apparatus according to item 1, characterized in that the reservoir means is a bottle for liquid recovery, and the bottle is rotatable in accordance with movement of the conduit.

(Item 5) The medical apparatus according to item 1, characterized in that an end face of the conduit that is connected is disposed at a position which is apart, by a predetermined distance, from a liquid level of the liquid that is recovered in the predetermined time.

The present invention is effective in the technical field of a medical apparatus which includes a pressure source and is used at a time of feeding/discharging air to/from a balloon that is attached to medical equipment such as an insertion tool for facilitating insertion of an endoscope into a body cavity, thereby inflating/deflating the balloon, and in the technical field in which this medical apparatus is manufactured and used.

## Claims

1. A medical apparatus (1) **characterized by** comprising:
medical equipment (3) which is provided with an opening end (24) for feeding/discharging a liquid;
a conduit (28) for feeding/discharging a fluid, which has one end communicating with the opening end of the medical equipment;
a pressure source (31) which generates a pressure difference for suction via the conduit (28);
a control unit (32) which controls a suction operation from the opening end (24) via the conduit (28) on the basis of the pressure difference that is generated by the pressure source (31), in accordance with an instruction from instruction means for instructing at least the suction operation from the opening end (24) of the medical equipment (3);
reservoir means (89) for recovering a liquid that moves in the conduit (28) in accordance with the suction operation based on a control of the control unit (32), the reservoir means communicating with the conduit (28) and having a preset predetermined capacity for storing the liquid; and
operation-canceling means for canceling the suction operation of sucking the liquid into the reservoir means (89), thereby to limit an amount of the recovered liquid in the reservoir means (89) within the predetermined capacity.

2. The medical apparatus (1) according to claim 1, **characterized in that** the medical equipment (3) includes an insertion section (21) which is inserted in a body cavity,
the insertion section (21) includes a balloon (26) which inflates/deflates in accordance with the pressure difference, and
the conduit (28) permits communication between the pressure source (31) and the balloon (26).

3. The medical apparatus (1) according to claim 1, **characterized in that** the operation-canceling means includes at least one of means for stopping the pressure source (31) after passing of a preset predetermined time, means for shutting the conduit (28) after the passing of the preset predetermined time, and means for leak to outside of the conduit (28) after the passing of the preset predetermined time.

4. The medical apparatus (1) according to claim 1, **characterized in that** the reservoir means (89) is a bottle (89) for liquid recovery,
a main body of the medical apparatus (1) includes a bottle holding section (94a, 94b) which rotatably holds the bottle (89), and
the bottle (89) is rotatable relative to the bottle holding section (94a, 94b) in accordance with movement of the conduit (28).

5. The medical apparatus (1) according to claim 4, **characterized in that** the bottle (89) is configured such that an end face of a connection part for connection to the conduit (28) is disposed at a position which is apart, by a predetermined distance, from a liquid level of the liquid that is recovered in a predetermined time.

6. The medical apparatus (1) according to claim 5, **characterized in that** the conduit (28) includes a medical-equipment-side conduit (28a) which is connected to an inner conduit (27) of the medical equipment (3), and a pressure-source-side conduit (28b) which is connected to the pressure source (31),
the bottle (89) includes a cylindrical bottle body (90),
the bottle holding section (94a, 94b) includes a C-shaped bottle holder (94a, 94b) which holds the bottle body (90) so as to be rotatable about a center axis of the bottle body (90) in a state in which the center axis of the bottle body (90) is set in a horizontal direction, and
the bottle body (90) is provided with a connection part (93) for connection to the pressure-source-side conduit (28b) at an upper end surface thereof, and a connection part (91) for connection to the medical-equipment-side conduit (28a) at an outer peripheral surface thereof.

7. The medical apparatus (1) according to claim 6, **characterized in that** the main body of the medical apparatus (1) includes a box-shaped outer case (69) which accommodates the pressure source (31) and the control unit (32),
the case (69) includes, on a front surface thereof, a concave-shaped bottle receiving recess portion (85) which receives the bottle (89), a mount portion (86) of the instruction means, and a led-out portion (87) of the pressure-source-side conduit (28b), and
the C-shaped bottle holder (94a, 94b) is mounted in the bottle receiving recess portion (85).

8. The medical apparatus (1) according to claim 6, **characterized in that** the bottle body (90) is configured such that an inner end portion of the connection part (93) for connection to the pressure-source-side conduit (28b) and an inner end portion of the connection part (91) for connection to the medical-equipment-side conduit (28a) are positioned above the liquid level of the liquid that is recovered in the bottle body (90) in the predetermined time.

9. The medical apparatus (1) according to claim 2, **characterized in that** the medical equipment (3) includes an insertion section (4) of an endoscope (2), and an overtube (3) which is provided over the insertion section (4) of the endoscope (2), and
the balloon (26) is provided over the overtube (3) .

10. The medical apparatus (1) according to claim 6, **characterized in that** the pressure source (31) is composed of a pump (31),
the pressure-source-side conduit (28b) includes at least an air feed flow path which causes a discharge pressure of the pump (31) to act on the bottle (89) side, and a suction flow path which causes a suction pressure of the pump (31) to act on the bottle (89) side, and
the control unit (32) includes switching means for effecting switching between the air feed flow path and the suction flow path in accordance with an instruction from the instruction means.

11. The medical apparatus (1) according to claim 7, **characterized in that** the instruction means includes at least a first controller (67) which is fixed to the case (69) of the main body of the medical apparatus, and a second controller which is a remote controller (68) that is connected to the main body of the medical apparatus.

12. The medical apparatus (1) according to claim 11, **characterized in that** the instruction means includes a third controller which is a foot switch (100) that is connected to the main body of the medical apparatus.

13. The medical apparatus (1) according to claim 6, **characterized in that** the pressure-source-side conduit (28b) includes a relief valve (V6) which is held in a closed state when a pressure in the conduit is lower than a preset pressure, and is opened when the pressure in the conduit becomes higher than the preset pressure, and
the relief valve (V6) is disposed at a position which is closest to the bottle (89) side in the pressure-source-side conduit (28b).

14. A medical apparatus (1) **characterized by** comprising:
medical equipment (3) which is provided with an opening end (24) for feeding/discharging a liquid;
a conduit (28) for feeding/discharging a fluid, which has one end communicating with the opening end of the medical equipment;
a pressure source (31) which generates a pressure difference for suction via the conduit (28);
a control unit (32) which controls a suction operation from the opening end (24) via the conduit (28) on the basis of the pressure difference that is generated by the pressure source (31), in accordance with an instruction from instruction unit (67,68) for instructing at least the suction operation from the opening end (24) of the medical equipment (3);
a bottle (89) for recovering a liquid that moves in the conduit (28) in accordance with the suction operation based on a control of the control unit (32), the bottle (89) communicating with the conduit (28) and having a preset predetermined capacity for storing the liquid; and
operation-canceling section (34) for canceling the suction operation of sucking the liquid into the bottle (89), thereby to limit an amount of the recovered liquid in the bottle (89) within the predetermined capacity.
